# EUROPEAN PATENT SPECIFICATION

(11) **EP 2 967 825 B1**
(45) Date of publication and mention of the grant of the patent: **25.01.2017**
(21) Application number: 14712391.3
(22) Date of filing: 25.02.2014
(51) Int. Cl.: A61F 2/07, A61F 2/88, A61F 2/915, A61L 31/00, B05C 13/02, B29D 23/00, B29C 53/00, B29C 65/00, B21F 45/00

(54) **METHODS AND APPARATUS FOR ASSEMBLING STENT-GRAFTS**
VERFAHREN UND VORRICHTUNG ZUR MONTAGE VON STENT-GRAFTS
MÉTHODES ET APPAREIL D'ASSEMBLAGE D'ENDOPROTHÈSES-GREFFONS

(30) Priority: 14.03.2013 US 201361784279 P; 21.02.2014 US 201414186935
(43) Date of publication of application: 20.01.2016
(73) Proprietor: W. L. Gore & Associates, Inc., Newark, DE 19711 (US)
(72) Inventor: ZUKOWSKI, Stanislaw, L., Newark, DE 19711 (US)
(74) Representative: HGF Limited
(86) International application number: PCT/US2014/018442
(87) International publication number: WO 2014/158580

(56) References cited:
- EP-A1- 1 719 538
- EP-A2- 0 893 108
- WO-A1-00/19942
- WO-A1-01/01887
- WO-A1-95/30385
- WO-A1-97/37617
- WO-A1-2010/111666
- WO-A2-00/42949
- WO-A2-98/26731
- WO-A2-2004/073557
- WO-A2-2008/027188
- US-A1- 2006 029 720
- US-A1- 2011 087 318
- US-B1- 6 245 099

## Description

### FIELD

The present disclosure relates generally to implantable medical device. More specifically, the present disclosure relates to methods and tooling for making flexible and comfortable stent grafts implantable in the vasculature of a patient.

### BACKGROUND

Implantable medical devices are frequently used to treat the anatomy of patients. Such devices can be permanently or semi-permanently implanted in the anatomy to provide treatment to the patient. In this regard, implantable devices such as stent-grafts can be implanted into the vasculature of a patient to provide a fluid bypass to a damaged area, such as an aneurysm.

EP 0 893 108 A2 discloses a stent-graft including a stent member having an inner surface and an outer surface, a generally tubular graft member and a coupling member in the form of a ribbon which covers only a portion of the inner or outer surface of the stent member and secures the stent member and graft member to one another. Alternatively, the coupling member can be described as interconnecting less than entirely the inner or outer surface of the graft member to the stent member. With this construction, regions of the stent member do not interfere with the coupling member.

WO 2008/027188 A2 discloses an implantable prosthesis, including a generally tubular substrate and a continuous shape memory member disposed over the outer surface of the substrate. The shape memory member may include a series of zig-zag struts alternating between a first strut with a first length and a second strut with a second length different from the first length. A graft member may be positioned over the substrate and shape memory member.

US 2011/087318 A1 discloses a conformable stent graft with an optional portal for a side branch device. Said stent graft comprises a graft being supported by a stent, wherein said stent comprises undulations each which comprise apices in opposing first and second directions and a tape member attached to said stent and to said graft such that the tape member edge is aligned to the edge of the apices in the first direction of the each of the undulations, thus confining the apices in the first direction of the undulations to the graft and wherein the apices in the second direction of the undulation are not confined relative to the graft; wherein said graft forms unidirectional pleats where longitudinally compressed and wherein said apices in the first direction of said undulation is positioned under an adjacent pleat when compressed. Also disclosed are methods of making and using said conformable stent graft and method of making the optional portal.

WO 01/01887 A1 discloses a composite stent/graft tubular prosthesis including an inner PTFE tubular structure, an outer PTFE tubular structure assembled about the inner PTFE tubular structure, and a circumferentially distensible stent interposed between the inner and outer PTFE tubular structures. The outer tubular body is a non-continuous body formed of polytetrafluoroethylene components, providing axial and circumferential compliance to said prosthesis. The outer tubular body completely overlies the distensible stent.

WO 95/30385 A1 discloses a stent comprising a self-expandable cylindrical body constructed of a single integral piece of material, including a plurality of wavy closed windings, and strips interconnecting the windings such that the stent is prevented from stretching longitudinally. A method of manufacture of the stent by winding a continuous wire upon spikes on an elongated mandrel to form a self-expanding shape memory coil is disclosed.

WO 2004/073557 A2 discloses a stent including a cylindrical frame consisting of a series of helical winds containing a pattern of alternating zigzag bends.

WO 2010/111666 A1 discloses vascular implants and methods for fabricating the same. The implantable devices include stents, grafts and stent grafts including one or more side branch lumens interconnected with the main lumen.

WO 00/19942 A1 discloses a flexible expandable sheet stent intended for the better support of a diseased vessel wall. The stent comprises constructive elements, preliminary formed in a shape of a stencil on the thin sheet metallic blank surface. The stent constructive elements include a saw-shaped profile made of teeth from which the relatively rigid band in a shape of consecutively united pockets is formed, the long twisting sides (9, 10) of the closed free loops and the short sides (11) of the closed ring outline. In a passage, formed of consecutively united pockets, a polymer thread (21) with a fixed length loaded with medicinal preparations for local drugs delivery is deployed.; The implantation of the stent in the vessel under the X-ray controls performed in such a way as to locate the relatively rigid band of consecutively united pockets on the side of the vessel wall adjoining the cardiac muscle.

WO 00/42949 A2 discloses an expandable stent with a covering of a material which is substantially impervious to body fluids and tissue ingrowth and has an increased resistance to bacterial attachment due to its lack of porosity and reduced surface texture. The resulting stent has a thin wall for minimum pre-deployment diameter and for minimum interference with fluid flow through the device after implantation. It has good flexibility, allowing its use in curved ducts.

US 6 245 099 B1 discloses a method for selectively bonding layers of polymeric material, especially expanded polytetrafluoroethylene (ePTFE), to create endoluminal vascular devices. The selective bonding is achieved by applying pressure to selected areas using a textured mandrel permitting a stent device to be encapsulated between two layers of ePTFE with unbonded slip pockets to accommodate movement of the structural members of the stent

WO 98/26731 A2 discloses a porous fluoropolymer article forming an artificial internal organ, for example, a vascular bypass, vascular access, or endovascular prosthesis. The prosthesis is unitary, but may be assembled in successive bodies which are coalesced, so that the porous microstructure changes distinctly at stages through the thickness dimension of the wall.

EP 1 719 538 A1 discloses a composite structure comprising two polytetrafluoroethylene porous layers and a framework structural member having a plurality of gaps or openings, the framework structural member being disposed between the two polytetrafluoroethylene porous layers, wherein the composite structure is structured such that the polytetrafluoroethylene porous layers are united together by being adhered with each other through the gaps or openings of the framework structural member and such that the respective polytetrafluoroethylene porous layers (A1) and (A2) are united with the framework structural member closely along the surfaces of the respective constituent elements of the framework structural member in such a manner as to wrap the respective elements. The method of manufacturing the composite structure is characterized in that it includes a step of applying pressure through a mass of fine particles.

US 2006/029720 A1 discloses methods and apparatus for coating at least a portion of the surface of medical devices using an injection coating device.

For example, stent-grafts are frequently deployed to areas in which they must conform to curved, angled, or otherwise non-linear vascular geometries. Such conformation requires the implantable device to be flexible yet resilient. Accordingly, there is a need for implantable medical devices, such as stent-grafts, with sufficient flexibility to conform to curved and/or angled vasculature geometries.

### BRIEF DESCRIPTION OF THE DRAWINGS

The accompanying drawings are included to provide a further understanding of the disclosure and are incorporated in and constitute a part of this specification, illustrate embodiments of the disclosure, and together with the description, serve to explain the principles of the disclosure, wherein;
Figure 1 illustrates a perspective view of a medical device in accordance with the present disclosure;
Figure 2 illustrates a side view of a segment of a medical device in accordance with the present disclosure;
Figure 3 illustrates a side view of a medical device in accordance with the present disclosure;
Figures 4A-4D illustrate cross-sectional end views of medical devices in accordance with the present disclosure;
Figures 5A-5D illustrate cross-sectional side views of medical devices in accordance with the present disclosure;
Figures 6A and 6B illustrate side views of mandrels for forming medical devices in accordance with the present disclosure;
Figures 7A-7F illustrate methods of forming a medical device in accordance with the present disclosure;
Figure 8 illustrates a side view of a segment of a medical device in accordance with the present disclosure;
Figure 9 illustrates a mandrel for forming a medical device in accordance with the present disclosure; and
Figure 10 illustrates a method for forming a medical device in accordance with the present disclosure

### DETAILED DESCRIPTION OF THE ILLUSTRATED EMBODIMENTS

Persons skilled in the art will readily appreciate that various aspects of the present disclosure can be realized by any number of methods and systems configured to perform the intended functions. Stated differently, other methods and systems can be incorporated herein to perform the intended functions. It should also be noted that the accompanying drawing figures referred to herein are not all drawn to scale, but can be exaggerated to illustrate various aspects of the present disclosure, and in that regard, the drawing figures should not be construed as limiting.

As used herein, "medical devices" can include, for example, stents, grafts, stent-grafts, filters, valves, occluders, markers, mapping devices, therapeutic agent delivery devices, prostheses, pumps, bandages, and other endoluminal and implantable devices that are implanted, acutely or chronically, in the vasculature or other body lumen or cavity at a treatment region. Such medical devices can comprise a flexible material that can provide a fluid-resistant or fluid-proof surface, such as a vessel bypass or blood occlusion.

The medical devices, support structures, coatings, and covers, described herein, can be biocompatible. As used herein, "biocompatible" means suited for and meeting the purpose and requirements of a medical device, used for either long- or short-term implants or for non-implantable applications. Long-term implants are generally defined as devices implanted for more than about 30 days.

As used herein, "membrane" means a layer of film or multiple layers of film concentrically arranged along a common axis to form a substantially tubular member.

For example, as described herein, a medical device such as a stent-graft can comprise a graft member comprising a flexible membrane that allows the stent-graft to be deployed in a blood vessel and provide a bypass route to avoid vessel damage or abnormalities, such as aneurysms. A stent member can be selectively attached to the graft member to provide increased flexibility of the resulting stent-graft. In accordance with an embodiment, the stent member is attached to the graft member in a manner that allows the stent member to curve and/or bend to accommodate the geometry of the treatment area of the patient.

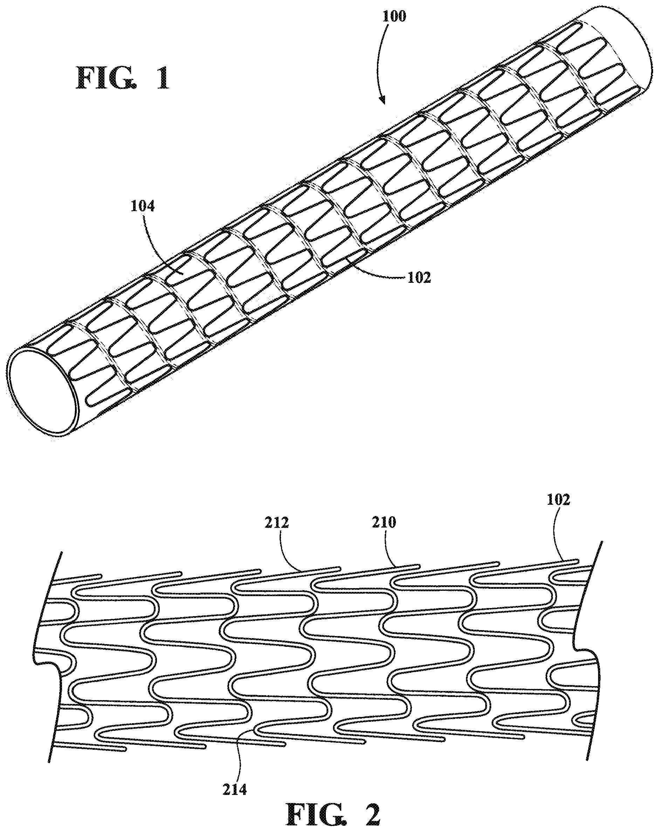

With reference now to Figure 1, a stent-graft 100 in accordance with the present disclosure is illustrated. Stent-graft 100 comprises a stent member 102 and a graft member 104. In various embodiments, graft member 104 is affixed to the outside surface of stent member 102, such that, once deployed, graft member 104 is in contact with a vessel wall. In other embodiments, graft member 104 is affixed to the inside surface of stent member 102, such that at least the stent member 102 is in contact with a vessel wall. In yet other embodiments, multiple graft members 104 can be utilized, such that one or more graft members 104 are affixed to the inner surface and one or more are affixed to the outer surface of stent member 102.

In various embodiments, stent member 102 comprises a biocompatible material. For example, stent member 102 can be formed from metallic, polymeric, or natural materials and can comprise conventional medical grade materials such as nylon, polyacrylamide, polycarbonate, polyethylene, polyformaldehyde, polymethylmethacrylate, polypropylene, polytetrafluoroethylene, polytrifluorochlorethylene, polyvinylchloride, polyurethane, elastomeric organosilicon polymers; metals such as stainless steels, cobalt-chromium alloys and nitinol, and biologically derived materials such as bovine arteries/veins, pericardium and collagen. Stent member 102 can also comprise bioresorbable materials such as poly(amino acids), poly(anhydrides), poly(caprolactones), poly(lactic/glycolic acid) polymers, poly(hydroxybutyrates) and poly(orthoesters). Any material which is biocompatible and provides adequate support for stent-graft 100 is in accordance with the present disclosure.

With reference to Figure 2, stent member 102 can comprise, for example, various configurations such as rings, cut tubes, wound wires (or ribbons), or flat patterned sheets rolled into a tubular form. In various embodiments, stent member 102 can comprise one or more wires formed into a pattern 210. For example, pattern 210 can comprise a helical pattern or a substantially sinusoidal pattern. In such configurations, pattern 210 comprises a series of alternating apices 214 and substantially straight segments 212. In various embodiments, stent member 102 can comprise a series of rings, wherein each ring is an individual wire manipulated to form pattern 210. Stent member 102 can also comprise a single wire manipulated to form pattern 210. However, any configuration of stent member 102 that can be implanted in and provide support to the vasculature of a patient is within the scope of the present disclosure.

With reference to Figure 1, in various embodiments, graft member 104 comprises a membrane of biocompatible material that provides a lumen for blood flow within a vasculature. For example, graft member 104 can comprise a composite material having a flexible matrix. In such configurations, the flexible matrix can comprise, for example, expanded polytetrafluoroethylene (ePTFE), pebax, polyester, polyurethane, fluoropolymers, such as perfouorelastomers and the like, polytetrafluoroethylene, silicones, urethanes, ultra high molecular weight polyethylene, aramid fibers, silk, and combinations thereof. Other flexible matrices can include high strength polymer fibers such as ultra high molecular weight polyethylene fibers (e.g., Spectra®, Dyneema Purity®, etc.) or aramid fibers (e.g., Technora®, etc.). Any graft member 104 that provides a sufficient lumen for blood flow within a vasculature is in accordance with the present disclosure.

Graft member 104 can comprise, for example, a tubular sheet of biocompatible material. In other embodiments, graft member 104 comprises a biocompatible tape wound to form a substantially tubular membrane. Further, graft member 104 can comprise one or more layers of material, including tubular sheets and/or windings of tape. Any configuration of graft member 104 that provides sufficient strength and flexibility is accordance with the present disclosure.

In various embodiments, graft member 104 can comprise an extruded flexible matrix. For example, graft member 104 can comprise an extruded ePTFE. In other embodiments, graft member 104 can comprise a composite material having a flexible matrix and an elastomeric component. An elastomeric component can comprise, for example, a perfluoroalkylvinylether (PAVE), such as perfluoromethylvinylether (PMVE), perfluoroethylvinylether (PEVE), or perfluoropropylvinylether (PPVE). Other biocompatible polymers which may be suitable for use in embodiments can include, but are not limited to, the group of urethanes, silicones, copolymers of silicon-urethane, styrene.isobutylene copolymers, polyisobutylene, polyethylene-co-poly(vinyl acetate), polyester copolymers, nylon copolymers, fluorinated hydrocarbon polymers and copolymers or mixtures of each of the foregoing. In such configurations, the flexible matrix is imbibed with the elastomeric component. Any elastomeric component that is biocompatible and can be imbibed by a suitable flexible matrix is in accordance with the present disclosure.

In various embodiments, stent-graft 100 comprises a stent member 102 selectively bonded to a portion of graft member 104. For example, a portion of stent member 102 can be bonded to graft member 104 in a manner designed to allow the resulting stent-graft 100 to curve, angle, and/or conform with the vasculature of a patient. In this regard, and as described in more detail herein, by selectively bonding stent member 102 to graft member 104, the non-bonded portion of stent member 102 can move or be displaced relative to graft member 104, which can provide the desired flexibility.

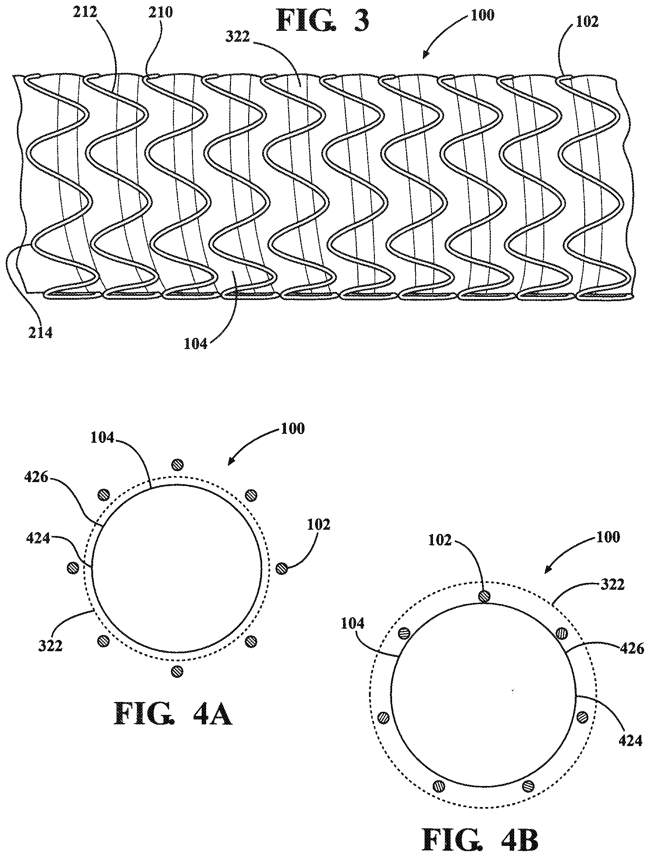

In various embodiments, and with reference to Figure 3, stent-graft 100 can be formed by surrounding graft member 104 with stent member 102 and connecting the two so that they maintain a desired orientation and positioning relative to each other. In various embodiments, graft member 104 comprises a bonding member 322. In such embodiments, bonding member 322 can contact a portion of stent member 102 to secure stent member 102 to the surface of graft member 104 and maintain the desired orientation and position between the two.

For example, as illustrated in Figure 3, bonding member 322 can comprise a layer of tape that adheres to the surface of graft member 104 and contacts a portion of stent member 102, such that the portion of stent member 102 is affixed to a surface of graft member 104. In such embodiments, bonding member 322 can comprise a thermoplastic material, such as FEP. In other embodiments, bonding member 322 can comprise a non-thermoplastic adhesive material.

As illustrated in Figure 3, bonding member 322 can be visually distinct from graft member 104. For example, bonding member 322 can comprise a color that contrasts with the color of graft member 104. This contrast can facilitate proper alignment of stent member 102 in relation to graft member 104 by providing a visual path or line to which stent member 102 can be aligned.

In various embodiments, bonding member 322 can comprise an adhesive material oriented helically with respect to graft member 104. In other embodiments, bonding member 322 can comprise one or more rings oriented substantially perpendicularly to a longitudinal axis of graft member 104, with consistent and/or variable spacing between each of the rings. In yet other embodiments, bonding member 322 can be oriented in one or more strips that are substantially parallel to the longitudinal axis of graft member 104, with consistent and/or variable spacing between each of the strips. Any combination of these orientations, as well as any other suitable orientation of bonding member 322 relative to graft member 104, is within the scope of the present disclosure.

In various embodiments, bonding member 322 selectively secures stent member 102 to graft member 104 by contacting and adhering to stent member 102 at a plurality of desired positions along pattern 210. Selectively securing stent member 102 can permit improved bending, curving, and/or conforming of stent-graft 100 to the vasculature of a patient. In conventional stent-grafts having a stent member wholly bonded to a graft member, the stent member is restricted from curving or bending by the graft member. Because the entire stent member is bonded to the graft member, the individual elements of the stent member (such as, for example, the apices) have a limited range of movement relative to the graft member. Although conventional graft members are typically somewhat flexible, the flexibility of the overall graft member is limited by the flexibility of the graft member. By selectively bonding the stent member to the graft member, the unbonded portion of the resulting stent-graft is capable of a greater degree of movement relative to the graft member. This increased relative movement can improve the bending, curving, and/or conforming capabilities of the stent graft. Further, the portion of the stent member selectively bonded to the graft member can be designed to allow for improved bending, curving, and/or conforming of the stent graft in a particular shape or direction. Some of the various manners in which stent member 102 is selectively bonded to graft member 104 will be discussed in greater detail below.

Bonding member 322 and stent member 102 can be aligned such that bonding member 322 contacts a plurality predetermined contact regions along the stent member 102. For example, as illustrated in Figure 3, the contact regions can include a plurality of substantially straight segments 212 of the stent member 102. Non-contact regions comprise portions of stent member 102 not in contact with bonding member 322 such as, for example, apices 214. In such embodiments, the non-contact regions of stent member 102 are afforded a greater degree of movement relative to the surface of graft member 104, which can facilitate bending, curving, and conforming of stent-graft 100 in the vasculature of a patient.

In other embodiments, one or more apices 214 can be in contact with and adhered by bonding member 322 to the surface of graft member 104, while other apices 214 are free from the surface of the graft member 104. In such embodiments, apices 214 which are not secured to the surface area of graft member 104 are afforded a greater degree of movement relative to the surface of graft member 104 than apices 214 which are secured, and the combination of secure and free apices 214 may allow for more a more specific bending, curving, and/or conforming of stent-graft 100. The particular apices 214 secured to the surface of graft member 104 by bonding member 322 can be chosen, for example, to allow stent-graft 100 to bend or curve more easily in a particular direction, and/or conform to a specific anatomy of a particular patient, such as, for example, an aortic arch of the patient.

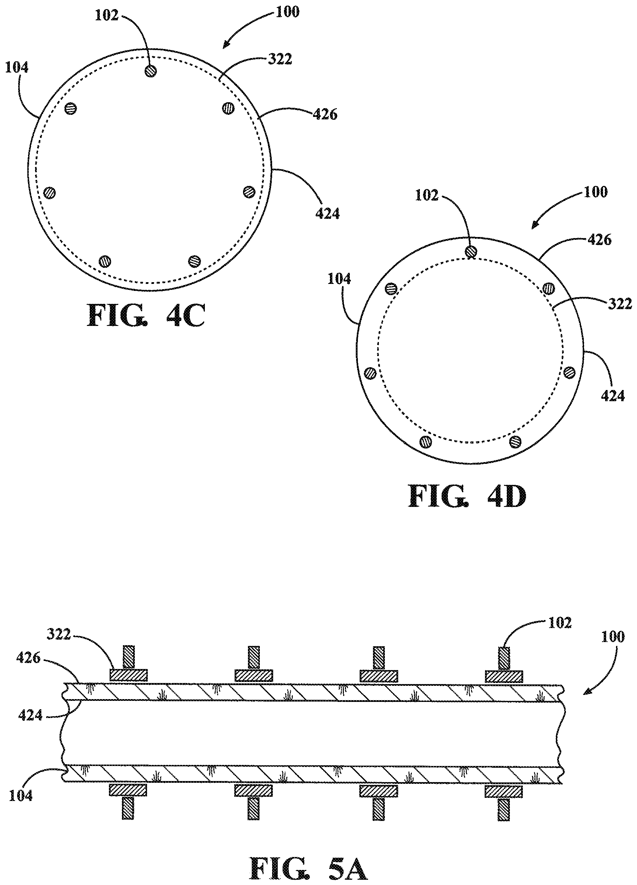

In various embodiments, and with reference to Figures 4A-4D and 5A-5D, stent member 102 and graft member 104 can be oriented relative to each other in a variety of ways. For example, with reference to Figures 4A and 5A, stent-graft 100 comprises stent member 102 concentrically surrounding graft member 104 and bonding member 322. In such configurations, bonding member 322 can comprise, for example, a tape or film disposed along an outer surface 426 of graft member 104, an adhesive applied to the outer surface 426 of graft member 104 in a desired pattern, or a layer of bonding material integrated into outer surface 426 of graft member 104.

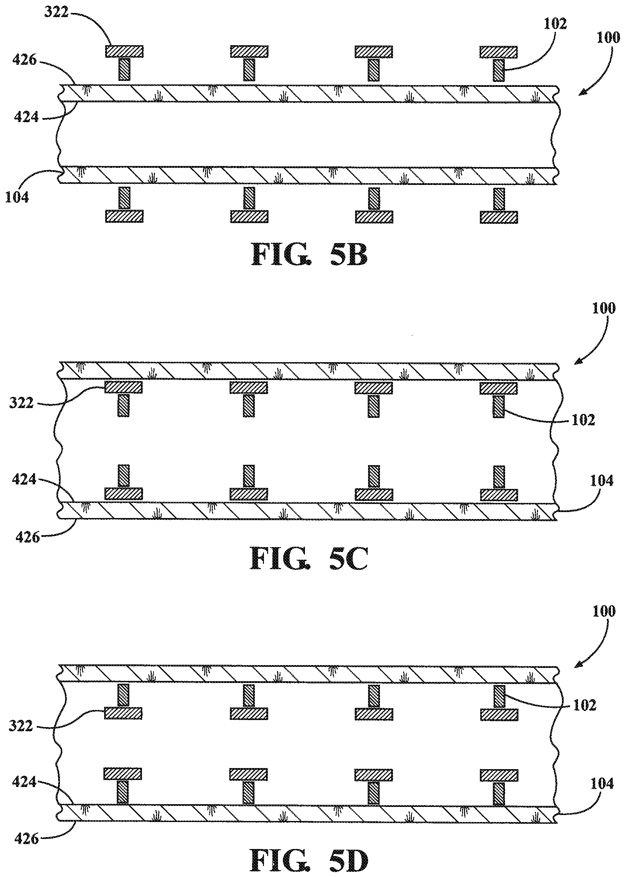

With reference to Figures 4B and 5B, stent-graft 100 comprises a stent member 102 concentrically surrounding graft member 104. Further, bonding member 322 surrounds a portion of stent member 102, securing the portion of stent member 102 to the outer surface of graft member 104. In such configurations, bonding member 322 can comprise, for example, an adhesive tape disposed circumferentially around stent member 102 in a desired pattern, or a tubular member having an adhesive disposed along a surface in a desired pattern.

In various embodiments, graft member 104 concentrically surrounds stent member 102. For example, with reference to Figures 4C and 5C, graft member 104 and bonding member 322 concentrically surround stent member 102, such that stent member 102 is in contact with an inner surface 424 of graft member 104. In such embodiments, bonding member 322 can comprise, for example, an adhesive applied to inner surface 424 of graft member 104 in a desired pattern, or a layer of bonding material integrated into inner surface 424 of graft member 104.

In other embodiments, as illustrated in Figures 4D and 5D, stent member 102 is concentrically surrounded by graft member 104. Stent member 102 concentrically surrounds bonding member 322, and bonding member contacts a portion of stent member 102, securing stent member 102 to inner surface 424 of graft member 104. In such embodiments, bonding member 322 can comprise, for example, a tubular member having an adhesive disposed along its outer surface in a desired pattern.

Although described in connection with a number of examples, including those illustrated in Figures 4A-4D and 5A-5D, any configuration in which a portion of stent member 102 is selectively secured to a surface, such as inner surface 424 or outer surface 426, of graft member 104 is in accordance with the present disclosure.

In various embodiments, including those described in relation to Figures 1, 3, 4A-4D and 5A-5D, stent member 102 can be selectively secured to graft member 104 in a temporary manner. For instance, bonding member 322 can comprise a material capable of temporarily securing portions of stent member 102 to a surface of graft member 104, creating a partially bonded stent-graft. In such configurations, and as will be discussed in greater detail herein, the partially bonded stent-graft can be used to form a fully bonded stent-graft 100 through further process steps.

In other embodiments, bonding member 322 can permanently secure a desired portion of stent member 102 to a surface of graft member 104. In such embodiments, bonding member 322 comprises a material capable of permanently securing portions of stent member 102 to a surface of graft member 104, creating a fully bonded stent-graft 100. Fully bonded stent-graft 100 can be utilized without further process steps to increase or improve the securement between stent member 102 and graft member 104.

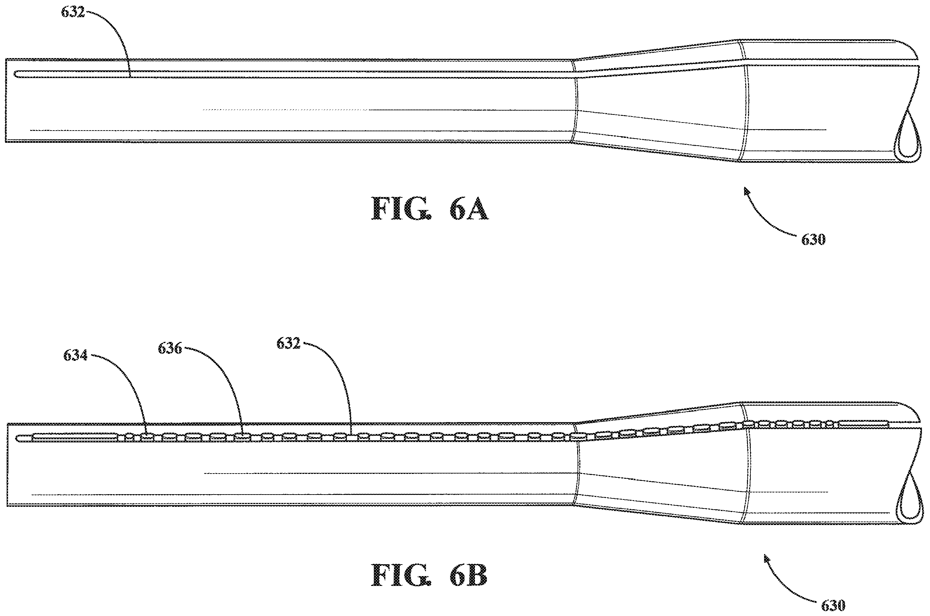

Stent-grafts, such as those illustrated in Figures 1, 3, 4A-4D, and 5A-5D, as well as others, can be formed by a variety of different methods. With reference to Figures 6A and 6B, a mandrel 630 can be used to form a variety of different stent-grafts 100. In various embodiments, mandrel 630 comprises a substantially tubular shape generally corresponding to a desired stent-graft shape. Mandrel 630, for example as illustrated in Figures 6A and 6B, can comprise a larger diameter portion and smaller diameter portion, and the portions can be connected by a tapered segment.

In various embodiments, mandrel 630 comprises any material capable of providing support to stent member 102 and graft member 104. The mandrel 630 can comprise metal, such as stainless steel, non-metals, or any suitable combination thereof.

In various embodiments, mandrel 630 comprises a tubular mandrel having a slot 632. Slot 632 can, for example, be oriented substantially parallel to a longitudinal axis of mandrel 630.

In various embodiments, mandrel 630 comprises an orientation member 634 to facilitate orientation of the stent relative to the graft and. Orientation member 634 can comprise, for example, a relatively thin and long metal member sized to fit within slot 632 of mandrel 630.

In various embodiments, orientation member 634 is movable along the slot 632. For example, orientation member 634 can be displaced relative to the outer surface of mandrel 630, such that orientation member 634 can be above or below the outer surface of mandrel 630. For example, mandrel 630 can be oriented such that slot 632 is generally radially oriented, and orientation member 634 can be generally radially translated within slot 632 above or below the outer surface of mandrel 630. In other embodiments, orientation member 634 can pivot such that a portion of orientation member 634 is above the outer surface of mandrel 630, and a second portion is below the outer surface of mandrel 630. Any manner of displacing orientation member 634 in relation to mandrel 630 and slot 632 is within the scope of the present disclosure.

Orientation member 634 can comprise, for example, a plurality of ribs 636. In various embodiments, the dimensions and spacing of the plurality of ribs 636 can correspond to the spacing of in a desired pattern 210 of stent member 102. For example, each of the plurality of ribs 636 can be spaced such that a portion of each of the ribs 636 corresponds with the desired position of apices 214 of pattern 210. In such embodiments, ribs 636 can be configured to interact with and hold specific apices of 214 in desired positions relative to graft member 104.

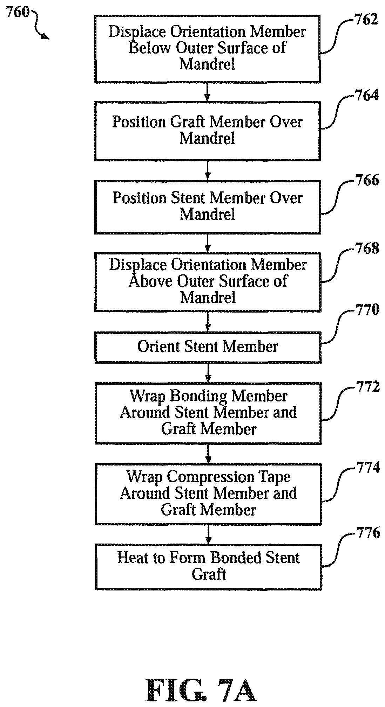

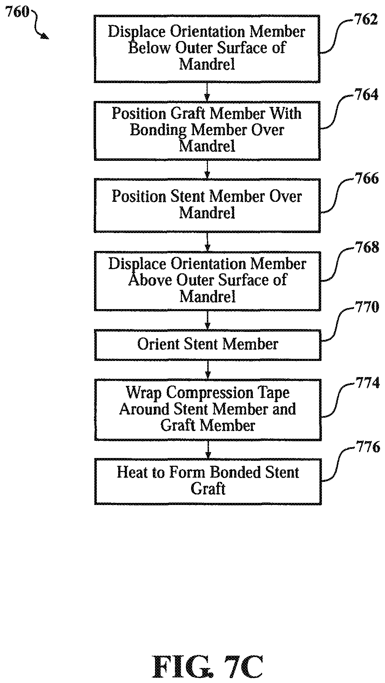

With reference to Figures 7A-7F, various methods for using mandrel 630 to form various stent-grafts 100 are illustrated. For example, Figure 7A illustrates a method 760 for forming a stent-graft, such as, for example, stent-graft 100 as illustrated in Figure 1. Method 760 comprises a displace orientation member below outer surface of mandrel step 762. In step 762, orientation member 634 is displaced such that ribs 636 are positioned within slot 632 and do not protrude above the outer surface of mandrel 630.

In various embodiments, method 760 further comprises a position graft member over mandrel step 764. In step 764, a graft member such as graft member 104 can be placed concentrically around mandrel 630. For example, graft member 104 can be positioned around mandrel 630 such that at least a portion of graft member 104 surrounds slot 632. In various embodiments, graft member 104 can comprise bonding member 322.

Method 760 can further comprise a position stent member over mandrel step 766. In various embodiments, step 766 comprises concentrically surrounding mandrel 630 and graft member 104 with stent member 102. For example, stent member 102 can be positioned around mandrel 630 such that at least a portion of stent member 102 surrounds slot 632.

In various embodiments, method 760 further comprises a displace orientation member above outer surface of mandrel step 768. In such embodiments, after both graft member 104 and stent member 102 have been positioned around mandrel 630, orientation member 634 is displaced such that ribs 636 are located above the outer surface of mandrel 630.

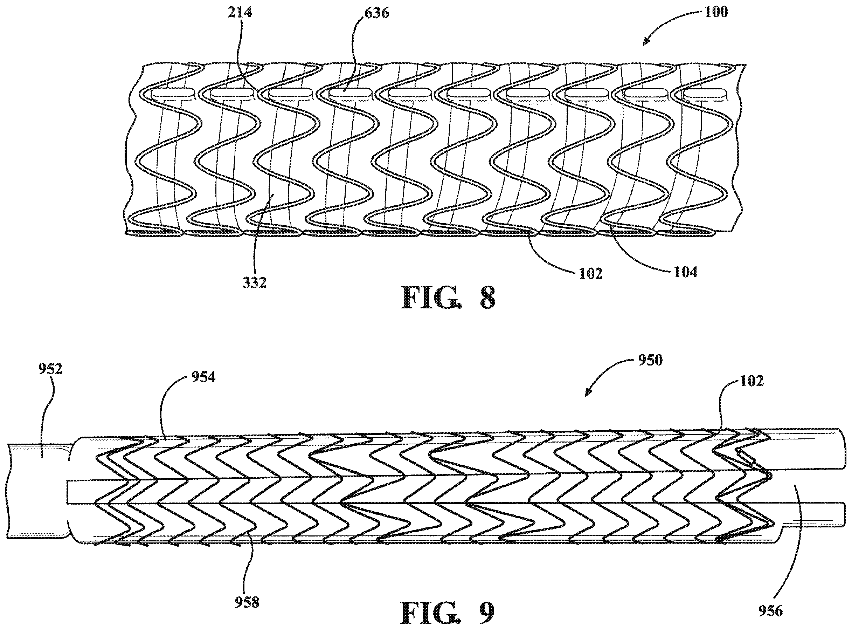

Method 760 can further comprise an orient stent member step 770. In various embodiments, step 770 comprises aligning stent member 102 in a desired position relative to graft member 104. For example, with reference to Figure 8, stent member 102 can be aligned such that apices 214 of pattern 210 are in contact with and held in a desired position by one or more of ribs 636. In such embodiments, ribs 636 provide proper spacing to stent member 102 which can assist in achieving the desired orientation between stent member 102 and graft member 104. In embodiments in which graft member 104 comprises bonding member 322, orientation member 634 of mandrel 630 can assist in orienting portions of pattern 210, such as straight segments 212, in a desired position relative to bonding member 322.

For example, Figure 8 illustrates a mandrel 630 with various components during step 770. Apices 214 of stent member 102 are aligned with ribs 636 of orientation member 634 to provide proper orientation of stent member 102 to bonding member 322. As illustrated, the proper positioning of apices 214 allows for bonding member 322 to contact straight segments 212 of stent member 102, as desired.

In various embodiments, method 760 further comprises a wrap bonding member around stent member and graft member step 772. For example, as illustrated in Figure 7A, bonding member 322 is wrapped around stent member 102 and graft member 104 in a pattern to selectively secure stent member 102 to graft member 104.

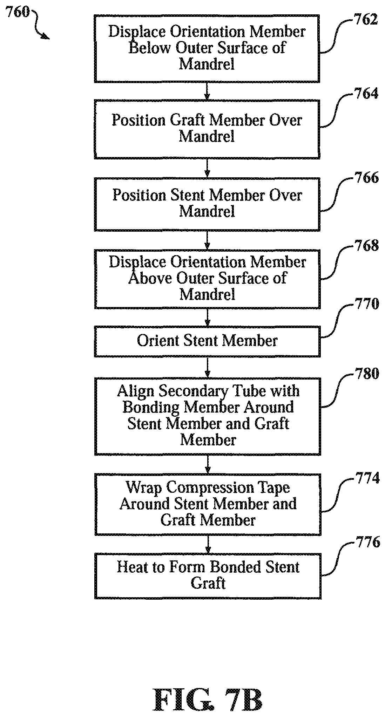

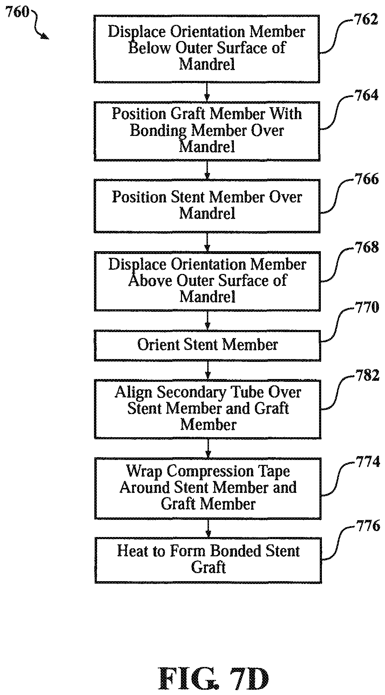

In other embodiments, as illustrated in Figures 7B and 7D, a secondary tube can be positioned around stent member 102 and graft member 104. For example, Figure 7D illustrates an align secondary tube over stent member and graft member step 782. In such embodiments, the secondary tube is positioned to concentrically surround and selectively secure stent member 102 and graft member 104. In other embodiments, as illustrated in figure 7B, method 760 comprises an align secondary tube with bonding member around stent member and graft member step 780. As in step 782, a secondary tube is positioned around stent member 102 and graft member 104. However, in step 780, bonding member 322 is disposed along the inner surface of the secondary tube.

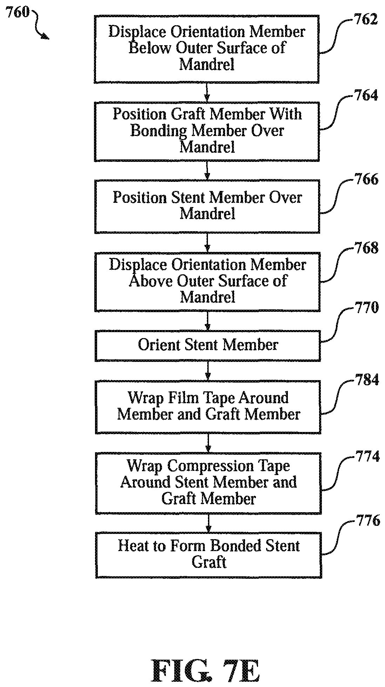

In yet other embodiments, and as illustrated in Figure 7E, method 760 comprises a wrap film tape around stent member and graft member step 784. In such embodiments, a film tape, such as, for example, ePTFE, is wrapped concentrically around stent member 102 and graft member 104. Step 784 can comprise, for example, surrounding stent member 102 with windings of film tape to help secure it to graft member 104.

With reference to Figures 7A-7E, method 760 further comprises a wrap compression tape around stent member and graft member step 774. Step 774 can, for example, comprise wrapping a compression tape around at least a portion of stent member 102 and graft member 104. In such configurations, the compression tape can provide, at least temporarily, a restrictive force against stent member 102 and graft member 104 and assist in maintaining proper alignment between the two components. Compression tape can comprise, for example, an ePTFE tape.

In various embodiments, method 760 comprises a heat to form bonded stent-graft step 776. Step 776, can comprise, for example, applying heat directly to mandrel 630, including stent member 102, graft member 104, and bonding member 322. Prior to applying heat to mandrel 630, step 776 can optionally include retracting orientation member 634 such that ribs 636 do not protrude above the outer surface of mandrel 630. During step 776, sufficient heat is applied to mandrel 630 to form a bonded stent-graft 100.

In various embodiments, step 776 can comprise applying heat to the entire mandrel 630 by, for example, placing mandrel 630 in an oven. In other embodiments, heat can be selectively applied to a portion of mandrel 630. For example, heat can be applied by a localized heat source, such as a torch or a heat gun, to the general area of slot 632 and orientation member 634. This localized application of heat can bond a portion of stent member 102 and graft member 104. However, any manner of applying heat to mandrel 630 to either selectively or wholly bond stent member 102 and graft member 104 is within the scope of the present disclosure.

After step 776 is complete, mandrel 630 and bonded stent-graft 100 can be permitted to cool to a suitably low temperature to allow for removal of the compression tape and disengagement of stent-graft 100 from mandrel 630.

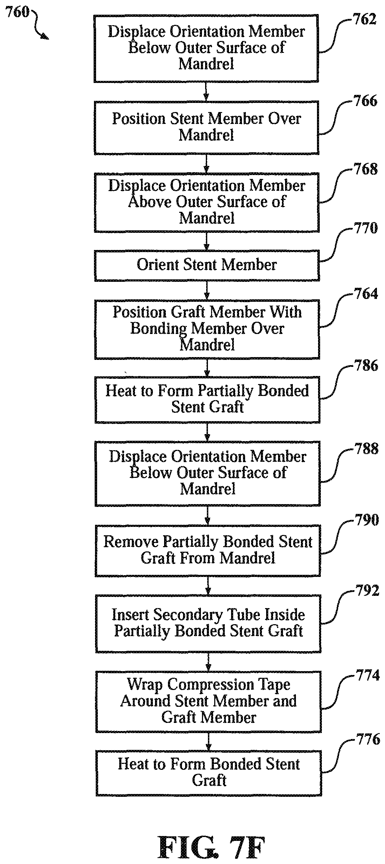

With reference to Figure 7F, an alternate method 760 for forming stent-graft 100 is illustrated. Method 760 comprises a number of the steps previously described in connection with Figures 7A-7E. As will be described below, these steps can be in a different chronological order than previously described embodiments. Further, additional steps are present in the method illustrated in Figure 7F.

In various embodiments, method 760 comprises a displace orientation member below outer surface of mandrel step 762, followed by a position stent member over mandrel step 766. In contrast to methods illustrated in Figures 7A-7E, stent member 102 is positioned over mandrel 630 before graft member 104.

Method 760 can comprise, for example the steps of displacing orientation member above outer surface of mandrel step 768 and orient stent member step 770. In various embodiments, position graft member with bonding member over mandrel step 764 occurs after orient stent member step 770.

Step 764 can comprise selecting an appropriate graft member 104 and bonding member 322. For example, graft member 104 can comprise a film tube, such as extruded ePTFE, and bonding member 322 can comprise a continuous coating of FEP. In other embodiments, graft member 104 comprises a film tube with cuts selectively placed to allow segments of apices 214 to protrude out of the cuts during bending and/or curving of stent-graft 100. In yet other embodiments, graft member 104 comprises a film tube, and bonding member 322 comprises an adhesive or thermoplastic applied to inner surface 424 of graft member 104 in a desired pattern. However, any graft member 104 and bonding member 322 capable of being partially bonded with and maintaining proper orientation relative to stent member 102 is within the scope of the present disclosure.

After step 764, method 760 can comprise a heat to form partially bonded stent-graft step 786. In such embodiments, heat is applied to the properly oriented stent member 102 and graft member 104 to partially or wholly bond the two and maintain the proper orientation. Further, in various embodiments, sufficient heat can be applied to mandrel 630 to temporarily bond stent member 102 and graft member 104. In other embodiments, sufficient heat is applied to mandrel 630 to permanently bond stent member 102 and graft member 104. Any manner in which heat is applied to mandrel 630 to partially or wholly, and temporarily or permanently, bond stent member 102 and graft member 104 is in accordance with the present disclosure.

Method 760 can further comprise a displace orientation member below outer surface of mandrel step 788 and a remove partially bonded stent-graft from mandrel step 790. Step 788 comprises displacing orientation member 634 such that plurality of ribs 636 are below the outer surface of mandrel 630. This can facilitate removal of the partially bonded stent-graft from mandrel 630, which can occur during step 790. After removal from mandrel 630, the partially bonded stent-graft can be implanted in the body of a patient or undergo further processing.

In various embodiments, method 760 comprises an insert secondary tube inside partially bonded stent-graft step 792. Step 792 can comprise, for example, inserting a biocompatible polymeric tube, such as a thin wall extruded ePTFE tube, inside of graft member 104, such that the partially bonded stent-graft concentrically surrounds the secondary tube. In various embodiments, a secondary mandrel is inserted inside of the secondary tube, and can provide a stable structure for further processing of the partially bonded stent-graft.

Method 760 can further comprise a wrap compression tape around stent member and graft member step 774 and heat to form bonded stent-graft step 776. As discussed in connection with Figures 7A-7E, a compression tape can be used to compress and maintain the proper orientation of stent member 102 and graft member 104. Heat can then be provided to permanently bond the two together, forming a suitable stent-graft 100.

Although particular methods for forming a stent-graft using mandrel 630 are illustrated in Figures 7A-7F and described herein, any method for producing an implantable stent-graft 100 using mandrel 630 is within the scope of the present disclosure.

The various-stent-grafts 100 illustrated above, and others, can also be formed using a slotted mandrel. For example, Figure 9 illustrates a mandrel 950 comprising two forks 954, a main body 952, and a slot 956. Slot 956 is a void between the inner surfaces of each fork 954. Forks 954 can be connected to main body 952 or otherwise elastically bendable so as to be movable relative to each other to facilitate placement and removal of stent, graft and/or stent grafts onto and from the forks 954. Moving forks 954 relative to each other changes the shape and dimensions of slot 956. For example, forks 954 can be displaced between a first position and a second position relative to each other.

Forks 954 are symmetrical. Forks 954 can be oriented, for example, substantially parallel to each other and to a longitudinal axis of mandrel 950. Forks 954 are non-parallel to each other and the longitudinal axis of mandrel 950. Any orientation of forks 954 is within the scope of the present disclosure.

Mandrel 950 further comprises a grooved section 958. Grooved section 958 is disposed along at least a portion of the outer surface of each fork 954 and comprises a plurality of grooves. Grooved section 958 can comprise a substantially sinusoidal pattern. In other configurations, grooved section 958 comprises a varied sinusoidal pattern, such that one or more pairs of adjacent apices vary in their proximity to one another. The configuration of grooved section 958 can, for example, correspond with a desired pattern 210 of stent member 102. In such configurations, stent member 102 can interact with grooved section 958 such that grooved section 958 aligns and maintains stent member 102 in a desired position relative to graft member 104. However, any desired configuration of grooved section 958 of mandrel 950 is within the scope of the present disclosure.

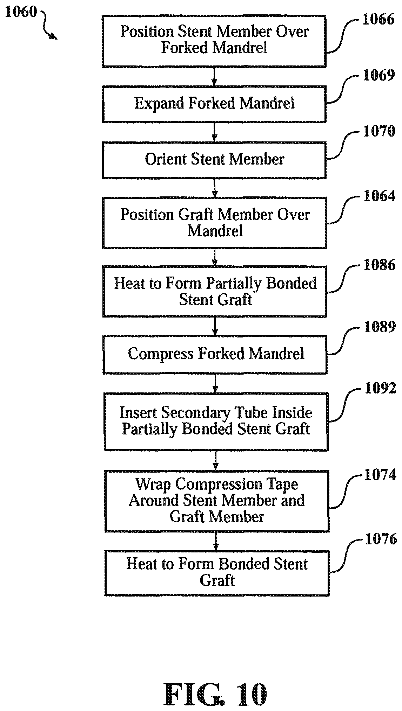

With reference to Figure 10, method 1060 comprises a method for forming stent-graft 100 using mandrel 950. Method 1060 comprises a position stent member over mandrel step 1066. Stent member 102 is positioned concentrically around forks 954.

Method 1060 can comprise an expand mandrel step 1070. Step 1070 comprises displacing forks 954 of mandrel 950 from a first to a second position, such that the distance between forks 954, and thus, the width of slot 956, is expanded.

Method 1060 can further comprise an orient stent member step 1070. Step 1070 comprises aligning stent pattern 210 such that at least one apex 214 is aligned with at least one of the plurality of grooves in grooved section 958.

After stent member 102 is properly aligned with respect to mandrel 950, method 1060 can comprise a position graft member over mandrel step 1064. Graft member 104 is positioned such that it concentrically surrounds forks 954 and stent member 102. Graft member 104 comprises bonding member 322. For example, graft member 104 can comprise a film tube, such as extruded ePTFE, and bonding member 322 can comprise a continuous coating of FEP. Graft member 104 comprises a film tube with cuts selectively placed to allow segments of apices 214 to protrude out of the cuts during bending and/or curving of stent-graft 100. Graft member 104 comprises a film tube, and bonding member 322 comprises an adhesive or thermoplastic applied to inner surface 424 of graft member 104 in a desired pattern. However, any graft member 104 and bonding member 322 capable of being partially bonded with and maintaining proper orientation relative to stent member 102 is within the scope of the present disclosure.

Method 1060 further comprises a heat to form partially bonded stent-graft step 1086. Heat is applied to mandrel 950, stent member 102, and graft member 104. For example, heat can be applied to stent member 102 and graft member 104 in selected regions such as the general region of slot 956, causing bonding member 322 to partially bond stent member 102 and graft member 104 along the surface of each where the heat was applied. By applying heat to these components in the region of slot 956, heat can be applied more precisely and dissipate more quickly, providing more predictable and precise points and/or areas of adhesion between stent member 102 and graft member 104 and reducing potential adherence of graft member 104 to mandrel 950.

Method 1060 can further comprise a compress mandrel step 1089. Step 1089 comprises displacing forks 954 of mandrel 950 from a second position to a first position, such that the distance between forks 954, and thus, the width of slot 956, is compressed. The partially bonded stent-graft can then be removed from mandrel 950.

Method 1060 further comprises an insert secondary tube inside partially bonded stent-graft step 1092. As discussed in connection Figure 7F and embodiments of method 760, step 1092 can comprise, for example, inserting a biocompatible polymeric tube, such as a thin wall extruded ePTFE tube, inside of graft member 104, such that the partially bonded stent-graft concentrically surrounds the secondary tube. A secondary mandrel is inserted inside of the secondary tube, and can provide a stable structure for further processing of the partially bonded stent-graft.

Similarly to the methods illustrated in Figures 7A-7F, method 1060 can further comprise a wrap compression tape around stent member and graft member step 1074 and heat to form bonded stent-graft step 1076. A compression tape can be used to compress and maintain the proper orientation of stent member 102 and graft member 104. Heat can then be provided to permanently bond the two together, forming a suitable stent-graft 100.

Although particular methods for forming a stent-graft using mandrel 950 are illustrated in Figure 10 and described herein, any method for producing an implantable stent-graft 100 using mandrel 950 is within the scope of the present disclosure.

In accordance with the present disclosure, in various embodiments, stent-graft 100 can comprise coatings. In various embodiments, the coatings comprise bio-active agents. Bio-active agents can be coated onto a portion or the entirety of the stent and/or graft member for controlled release of the agents once the device is implanted. The bio-active agents can include, but are not limited to, vasodilator, anti-coagulants, such as, for example, warfarin and heparin. Other bio-active agents can also include, but are not limited to agents such as, for example, anti-proliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) IIb/IIIa inhibitors and vitronectin receptor antagonists; anti-proliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes-dacarbazinine (DTIC); anti-proliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; anti-sense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

Stent-graft 100 can be deployed using any suitable device delivery system. The device delivery system can comprise one or more catheters, guidewires, or other suitable conduits for delivering an elongated segment to a treatment region. The catheters, guidewires, or conduits can comprise lumens configured to receive inputs and/or materials from the proximal end of the medical device delivery system and conduct the inputs and/or materials to the elongated segment at the treatment region. Further, stent-graft 100 can comprise a self-expandable or a balloon expandable implant.

It will be apparent to those skilled in the art that various modifications and variations can be made in the present disclosure without departing from the scope of the disclosure. Thus, it is intended that the present disclosure cover the modifications and variations of this disclosure provided they come within the scope of the appended claims and their equivalents.

Likewise, numerous characteristics and advantages have been set forth in the preceding description, including various alternatives together with details of the structure and function of the devices and/or methods. The disclosure is intended as illustrative only and as such is not intended to be exhaustive. It will be evident to those skilled in the art that various modifications can be made, especially in matters of structure, materials, elements, components, shape, size and arrangement of parts including combinations within the principles of the disclosure, to the full extent indicated by the broad, general meaning of the terms in which the appended claims are expressed. To the extent that these various modifications do not depart from the scope of the appended claims, they are intended to be encompassed therein.

## Claims

1. A method of making a device (100) comprising:
providing a substantially tubular mandrel (630) having an outer surface and a longitudinal axis, wherein the mandrel (630) includes a slot (632) extending through the outer surface along the longitudinal axis, and an orientation member (634) disposed within the slot (632), wherein the orientation member (634) is perpendicularly displaceable within the slot (632) relative to the outer surface of the tubular mandrel (630);
displacing the orientation member (634) below the outer surface of the mandrel (630);
positioning a graft member (104) over the mandrel (630);
positioning a stent member (102) over the mandrel (630);
displacing the orientation member (634) above the outer surface of the mandrel 630); and
orienting the stent member (102) relative to the orientation member (634).

2. The method of claim 1, wherein the graft member (104) concentrically surrounds at least a portion of the stent member (102); and optionally
the graft member (104) further comprises a bonding member (322) disposed along at least a portion of an outside surface of the graft member (104).

3. The method of claim 1, wherein the stent member (102) concentrically surrounds at least a portion of the graft member (104); and optionally
the graft member (104) further comprises a bonding member (322) disposed along at least a portion of an inner surface of the graft member (104).

4. The method of claim 1, wherein the graft member (104) concentrically surrounds at least a portion of the stent member (102); and the graft member (104) further comprises a bonding member (322) disposed along at least a portion of an outside surface of the graft member (104); and further comprising a step of wrapping a film tape over at least as portion of the outer surface of the stent member (102); or
further comprising a step of surrounding at least a portion of the outer surface of the stent member (102) with a secondary tube; and optionally
further comprising a step of applying heat to at least a portion of the mandrel (630) to fixedly secure the position of the stent member (102) relative to the graft member (104) to form a partially bonded stent-graft (100); and
wherein heat is applied to the orientation member (634) of the mandrel (630).

5. The method of claim 1, wherein the graft member (104) concentrically surrounds at least a portion of the stent member (102);
the graft member (104) further comprises a bonding member (322) disposed along at least a portion of an outside surface of the graft member (104);
further comprising a step of surrounding at least a portion of the outer surface of the stent member (102) with a secondary tube;
further comprising a step of applying heat to at least a portion of the mandrel (630) to fixedly secure the position of the stent member (102) relative to the graft member (104) to form a partially bonded stent-graft (100);
wherein heat is applied to the orientation member (634) of the mandrel (630); and
further comprising the steps of:
removing the partially bonded stent-graft (100) from the mandrel (630);
inserting a secondary tube inside the inner surface of the partially bonded stent-graft (100);
inserting a secondary mandrel inside the secondary tube;
wrapping a compression tape concentrically around the partially bonded stent-graft (100); and
applying heat to at least a portion of the secondary mandrel to form a bonded stent-graft (100).

6. The method of claim 1, wherein the graft member (104) concentrically surrounds at least a portion of the stent member (102);
the graft member (104) further comprises a bonding member (322) disposed along at least a portion of an outside surface of the graft member (104);
further comprising a step of surrounding at least a portion of the outer surface of the stent member (102) with a secondary tube;
further comprising a step of applying heat to at least a portion of the mandrel (630) to fixedly secure the position of the stent member (102) relative to the graft member (104) to form a partially bonded stent-graft (100);
wherein heat is applied to the orientation member (634) of the mandrel (630); further comprising the steps of:
wrapping a compression tape concentrically around the partially bonded stent-graft (100); and
applying heat to the mandrel (630) to form a bonded stent-graft (100).

7. The method of claim 5, wherein the graft member (104) comprises a film tube, wherein the bonding member (322) comprises a layer of thermoplastic disposed along the inner surface of the film tube, and wherein the graft member (104) comprises a plurality of cuts; and optionally
wherein the graft member (104) comprises an ePTFE tube; or
wherein the secondary tube comprises an ePTFE tube; and optionally wherein the bonding member (322) comprises FEP; and
wherein the stent member (102) comprises a single wire in a helical pattern and a plurality of apices (214); or
wherein the stent member (102) comprises a plurality of rings, each ring having a substantially sinusoidal pattern and a plurality of apices (214); and optionally
wherein the orientation member (634) comprises a plurality of ribs (636).

8. The method of claim 5, wherein the secondary tube comprises an ePTFE tube;
the bonding member (322) comprises FEP;
the stent member (102) comprises a plurality of rings, each ring having a substantially sinusoidal pattern and a plurality of apices (214);
the orientation member (634) comprises a plurality of ribs (636);
wherein the step of orienting the stent member (102) relative to the graft member (104) comprises positioning the stent member (102) relative to the orientation member (634) such that one or more apices (214) of the stent member (102) are in contact with the one or more ribs (636) of the orientation member (634); and optionally
wherein the stent member (102) comprises nitinol; or
wherein each of the plurality of ribs (636) comprises a width, the width being substantially the same for each of the plurality of ribs (636); and optionally
wherein the width of at least one of the plurality of ribs (636) is equal to the distance between two adjacent apices (214) of the stent member (102).

9. An article for manufacturing a stent-graft (100) comprising:
a substantially tubular mandrel (630) comprising an outer surface, a longitudinal axis, a slot (632), and an orientation member (634),
wherein the orientation member (634) is located within the slot (632) and is substantially parallel to the longitudinal axis of the tubular mandrel (630), and is capable of being perpendicularly displaced relative to the outer surface of the tubular mandrel (630), and
wherein the orientation member (634) comprises a plurality of ribs (636), and wherein the position of each of the plurality of ribs (636) correlates to a desired stent pattern (210).

10. The article of claim 9, wherein the stent pattern (210) comprises a substantially helical pattern; or
wherein each of the plurality of ribs (636) comprises a width, the width being substantially the same for each of the plurality of ribs (636); or
wherein each of the plurality of ribs (636) comprises a width, the width varying between at least two of the plurality of ribs (636).

## Patentansprüche

1. Verfahren zum Herstellen einer Vorrichtung (100), Folgendes umfassend:
Bereitstellen einer im Wesentlichen röhrenförmigen Spindel (630) mit einer Außenfläche und einer Längsachse, wobei die Spindel (630) einen Schlitz (632) enthält, der sich durch die Außenfläche entlang der Längsachse erstreckt, und ein Ausrichtungselement (634), das innerhalb des Schlitzes (632) angebracht ist, wobei das Ausrichtungselement (634) innerhalb des Schlitzes (632) relativ zur Außenfläche der röhrenförmigen Spindel (630) senkrecht verschiebbar ist;
Verschieben des Ausrichtungselements (634) unter die Außenfläche der Spindel (630);
Positionieren eines Transplantatelements (104) über der Spindel (630);
Positionieren eines Stentelements (102) über der Spindel (630);
Verschieben des Ausrichtungselements (634) über die Außenfläche der Spindel (630); und
Ausrichten des Stentelements (102) relativ zu dem Ausrichtungselement (634).

2. Verfahren nach Anspruch 1, wobei das Transplantatelement (104) wenigstens einen Abschnitt des Stentelements (102) konzentrisch umgibt; und wobei
das Transplantatelement (104) gegebenenfalls weiter ein Bindungselement (322) umfasst, das entlang wenigstens eines Abschnitts einer Außenfläche des Transplantatelements (104) angebracht ist.

3. Verfahren nach Anspruch 1, wobei das Stentelement (102) wenigstens einen Abschnitt des Transplantatelements (104) konzentrisch umgibt; und wobei
das Transplantatelement (104) gegebenenfalls weiter ein Bindungselement (322) umfasst, das entlang wenigstens eines Abschnitts einer Innenfläche des Transplantatelements (104) angebracht ist.

4. Verfahren nach Anspruch 1, wobei das Transplantatelement (104) wenigstens einen Abschnitt des Stentelements (102) konzentrisch umgibt; und das Transplantatelement (104) weiter ein Bindungselement (322) umfasst, das entlang wenigstens eines Abschnitts einer Außenfläche des Transplantatelements (104) angebracht ist; und weiter umfassend einen Schritt des Wickelns eines Folienbandes über wenigstens einen Abschnitt der Außenfläche des Stentelements (102); oder
weiter umfassend einen Schritt des Umgebens wenigstens eines Abschnitts der Außenfläche des Stentelements (102) mit einer sekundären Röhre; und gegebenenfalls
weiter umfassend einen Schritt des Anwendens von Wärme auf wenigstens einen Abschnitt der Spindel (630), um die Position des Stentelements (102) relativ zu dem Transplantatelement (104) stabil zu festigen, um ein teilweise gebundenes Stenttransplantat (100) auszubilden; und
wobei Wärme auf das Ausrichtungselement (634) der Spindel (630) angewandt wird.

5. Verfahren nach Anspruch 1, wobei das Transplantatelement (104) wenigstens einen Abschnitt des Stentelements (102) konzentrisch umgibt;
das Transplantatelement (104) gegebenenfalls weiter ein Bindungselement (322) umfasst, das entlang wenigstens eines Abschnitts einer Außenfläche des Transplantatelements (104) angebracht ist;
weiter umfassend einen Schritt des Umgebens wenigstens eines Abschnitts der Außenfläche des Stentelements (102) mit einer sekundären Röhre;
weiter umfassend einen Schritt des Anwendens von Wärme auf wenigstens einen Abschnitt der Spindel (630), um die Position des Stentelements (102) relativ zu dem Transplantatelement (104) stabil zu festigen, um ein teilweise gebundenes Stenttransplantat (100) auszubilden;
wobei Wärme auf das Ausrichtungselement (634) der Spindel (630) angewandt wird; und weiter umfassend die folgenden Schritte:
Entfernen des teilweise gebundenen Stenttransplantats (100) von der Spindel (630); Einführen einer sekundären Röhre in die Innenfläche des teilweise gebundenen Stenttransplantats (100);
Einführen einer sekundären Spindel in die sekundäre Röhre;
konzentrisches Wickeln eines Kompressionsbandes um das teilweise gebundene Stenttransplantat (100); und
Anwenden von Wärme auf wenigstens einen Abschnitt der sekundären Spindel, um ein gebundenes Stenttransplantat (100) auszubilden.

6. Verfahren nach Anspruch 1, wobei das Transplantatelement (104) wenigstens einen Abschnitt des Stentelements (102) konzentrisch umgibt;
das Transplantatelement (104) gegebenenfalls weiter ein Bindungselement (322) umfasst, das entlang wenigstens eines Abschnitts einer Außenfläche des Transplantatelements (104) angebracht ist;
weiter umfassend einen Schritt des Umgebens wenigstens eines Abschnitts der Außenfläche des Stentelements (102) mit einer sekundären Röhre;
weiter umfassend einen Schritt des Anwendens von Wärme auf wenigstens einen Abschnitt der Spindel (630), um die Position des Stentelements (102) relativ zu dem Transplantatelement (104) stabil zu festigen, um ein teilweise gebundenes Stenttransplantat (100) auszubilden;
wobei Wärme auf das Ausrichtungselement (634) der Spindel (630) angewandt wird; weiter umfassend die folgenden Schritte:
konzentrisches Wickeln eines Kompressionsbandes um das teilweise gebundene Stenttransplantat (100); und
Anwenden von Wärme auf die Spindel (630), um ein gebundenes Stenttransplantat (100) auszubilden.

7. Verfahren nach Anspruch 5, wobei das Transplantatelement (104) eine Folienröhre umfasst, wobei das Bindungselement (322) eine Lage von Thermoplast umfasst, die entlang der Innenfläche der Folienröhre angebracht ist, und wobei das Transplantatelement (104) eine Vielzahl von Schnitten umfasst; und
wobei das Transplantatelement (104) gegebenenfalls eine ePTFE-Röhre umfasst, oder
wobei die sekundäre Röhre eine ePTFE-Röhre umfasst; und
wobei das Bindungselement (322) gegebenenfalls FEP umfasst; und
wobei das Stentelement (102) einen einzelnen Draht in einem spiralförmigen Muster und eine Vielzahl von Spitzen (214) umfasst; oder
wobei das Stentelement (102) eine Vielzahl von Ringen umfasst, wobei jeder Ring ein im Wesentlichen sinusförmiges Muster und eine Vielzahl von Spitzen (214) aufweist; und wobei das Ausrichtungselement (634) gegebenenfalls eine Vielzahl von Rippen (636) umfasst.

8. Verfahren nach Anspruch 5, wobei die sekundäre Röhre eine ePTFE-Röhre umfasst; das Bindeelement (322) FEP umfasst;
das Stentelement (102) eine Vielzahl von Ringen umfasst, wobei jeder Ring ein im Wesentlichen sinusförmiges Muster und eine Vielzahl von Spitzen (214) aufweist;
das Ausrichtungselement (634) eine Vielzahl von Rippen (636) umfasst;
wobei der Schritt des Orientierens des Stentelements (102) relativ zu dem Transplantatelement (104) das Positionieren des Stentelements (102) relativ zu dem Ausrichtungselement (634) umfasst, so dass eine oder mehrere Spitzen (214) des Stentelements (102) mit der einen oder den mehreren Rippen (636) des Ausrichtungselements (634) in Kontakt sind; und
wobei das Stentelement (102) gegebenenfalls Nitinol umfasst; oder
wobei jede aus der Vielzahl von Rippen (636) eine Breite umfasst, wobei die Breite für jede aus der Vielzahl von Rippen (636) im Wesentlichen die gleiche ist; und
wobei die Breite wenigstens einer aus der Vielzahl von Rippen (636) gegebenenfalls dem Abstand zwischen zwei benachbarten Spitzen (214) des Stentelements (102) entspricht.

9. Gegenstand zum Erzeugen eines Stenttransplantats (100), Folgendes umfassend:
eine im Wesentlichen röhrenförmige Spindel (630), die eine Außenfläche, eine Längsachse, einen Schlitz (632) und ein Ausrichtungselement (634) umfasst,
wobei das Ausrichtungselement (634) innerhalb des Schlitzes (632) angebracht ist und im Wesentlichen parallel zur Längsachse der röhrenförmigen Spindel (630) verläuft und relativ zur Außenfläche der röhrenförmigen Spindel (630) senkrecht verschoben werden kann, und
wobei das Ausrichtungselement (634) eine Vielzahl von Rippen (636) umfasst, und wobei die Position jeder aus der Vielzahl von Rippen (636) mit einem gewünschten Stentmuster (210) korreliert.

10. Gegenstand nach Anspruch 9, wobei das Stentmuster (210) ein im Wesentlichen spiralförmiges Muster umfasst; oder
wobei jede aus der Vielzahl von Rippen (636) eine Breite umfasst, wobei die Breite für jede aus der Vielzahl von Rippen (636) im Wesentlichen die gleiche ist; oder
wobei jede aus der Vielzahl von Rippen (636) eine Breite umfasst,
wobei die Breite zwischen wenigstens zwei aus der Vielzahl von Rippen (636) variiert.

## Revendications

1. Procédé de fabrication d'un dispositif (100), comprenant :
l'utilisation d'un mandrin sensiblement tubulaire (630) comportant une surface extérieure et un axe longitudinal, dans lequel le mandrin (630) comprend une fente (632) s'étendant à travers la surface extérieure le long de l'axe longitudinal, et un élément d'orientation (634) disposé à l'intérieur de la fente (632), dans lequel l'élément d'orientation (634) peut être déplacé perpendiculairement à l'intérieur de la fente (632) par rapport à la surface extérieure du mandrin tubulaire (630) ;
le déplacement de l'élément d'orientation (634) au-dessous de la surface extérieure du mandrin (630) ;
le positionnement d'un élément greffe (104) sur le mandrin (630) ;
le positionnement d'un élément endoprothèse vasculaire (102) sur le mandrin (630) ;
le déplacement de l'élément d'orientation (634) au-dessus de la surface extérieure du mandrin (630) ; et
l'orientation de l'élément endoprothèse vasculaire (102) par rapport à l'élément d'orientation (634).

2. Procédé selon la revendication 1, dans lequel l'élément greffe (104) entoure concentriquement au moins une partie de l'élément endoprothèse vasculaire (102); et éventuellement
l'élément greffe (104) comprend en outre un élément de liaison (322) disposé le long d'au moins une partie d'une surface extérieure de l'élément greffe (104).

3. Procédé selon la revendication 1, dans lequel l'élément endoprothèse vasculaire (102) entoure concentriquement au moins une partie de l'élément greffe (104) ; et éventuellement
l'élément greffe (104) comprend en outre un élément de liaison (322) disposé le long d'au moins une partie d'une surface intérieure de l'élément greffe (104).

4. Procédé selon la revendication 1, dans lequel l'élément greffe (104) entoure concentriquement au moins une partie de l'élément endoprothèse vasculaire (102) ; et l'élément greffe (104) comprend en outre un élément de liaison (322) disposé le long d'au moins une partie d'une surface extérieure de l'élément greffe (104) ; et comprenant en outre une étape consistant à enrouler une bande de film sur au moins une partie de la surface extérieure de l'élément endoprothèse vasculaire (102) ; ou
comprenant en outre une étape consistant à entourer au moins une partie de la surface externe de l'élément endoprothèse vasculaire (102) d'un tube secondaire ; et éventuellement
comprenant en outre une étape consistant à appliquer de la chaleur à au moins une partie du mandrin (630) de façon à fixer définitivement la position de l'élément endoprothèse vasculaire (102) par rapport à l'élément greffe (104) pour former une endoprothèse vasculaire-greffe partiellement liée (100) ; et
dans lequel de la chaleur est appliquée à l'élément d'orientation (634) du mandrin (630).

5. Procédé selon la revendication 1, dans lequel l'élément greffe (104) entoure concentriquement au moins une partie de l'élément endoprothèse vasculaire (102) ;
l'élément greffe (104) comprend en outre un élément de liaison (322) disposé le long d'au moins une partie d'une surface extérieure de l'élément greffe (104) ;
comprenant en outre une étape consistant à entourer au moins une partie de la surface externe de l'élément endoprothèse vasculaire (102) d'un tube secondaire ;
comprenant en outre une étape consistant à appliquer de la chaleur à au moins une partie du mandrin (630) de façon à fixer définitivement la position de l'élément endoprothèse vasculaire (102) par rapport à l'élément greffe (104) pour former une endoprothèse vasculaire-greffe partiellement liée (100) ;
dans lequel de la chaleur est appliquée à l'élément d'orientation (634) du mandrin (630) ; et
comprenant en outre les étapes consistant à :
retirer l'endoprothèse vasculaire-greffe partiellement liée (100) du mandrin (630) ;
insérer un tube secondaire à l'intérieur de la surface intérieure de l'endoprothèse vasculaire-greffe partiellement liée (100) ;
insérer un mandrin secondaire à l'intérieur du tube secondaire ;
enrouler une bande de compression concentriquement autour de l'endoprothèse vasculaire-greffe partiellement liée (100) ; et
appliquer de la chaleur à au moins une partie du mandrin secondaire pour former une endoprothèse vasculaire-greffe liée (100).

6. Procédé selon la revendication 1, dans lequel l'élément greffe (104) entoure concentriquement au moins une partie de l'élément endoprothèse vasculaire (102) ;
l'élément greffe (104) comprend en outre un élément de liaison (322) disposé le long d'au moins une partie d'une surface extérieure de l'élément greffe (104) ;
comprenant en outre une étape consistant à entourer au moins une partie de la surface externe de l'élément endoprothèse vasculaire (102) d'un tube secondaire ;
comprenant en outre une étape consistant à appliquer de la chaleur à au moins une partie du mandrin (630) de façon à fixer définitivement la position de l'élément endoprothèse vasculaire (102) par rapport à l'élément greffe (104) pour former une endoprothèse vasculaire-greffe partiellement liée (100) ;
dans lequel de la chaleur est appliquée à l'élément d'orientation (634) du mandrin (630) ; comprenant en outre les étapes consistant à :
enrouler une bande de compression concentriquement autour de l'endoprothèse vasculaire-greffe partiellement liée (100) ; et
appliquer de la chaleur au mandrin (630) pour former une endoprothèse vasculaire-greffe liée (100).

7. Procédé selon la revendication 5, dans lequel l'élément greffe (104) comprend un tube de film, dans lequel l'élément de liaison (322) comprend une couche de thermoplastique disposée le long de la surface intérieure du tube de film, et dans lequel l'élément greffe (104) comprend plusieurs découpes ; et éventuellement
dans lequel l'élément greffe (104) comprend un tube en ePTFE ; ou
dans lequel le tube secondaire comprend un tube en ePTFE ; et éventuellement dans lequel l'élément de liaison (322) comprend du FEP ; et
dans lequel l'élément endoprothèse vasculaire (102) comprend un unique fil à motif hélicoïdal et plusieurs sommets (214) ; ou
dans lequel l'élément endoprothèse vasculaire (102) comprend plusieurs anneaux, chaque anneau ayant un motif sensiblement sinusoïdal et comportant plusieurs sommets (214); et éventuellement dans lequel l'élément d'orientation (634) comprend plusieurs nervures (636).

8. Procédé selon la revendication 5, dans lequel le tube secondaire comprend un tube en ePTFE ; l'élément de liaison (322) comprend du FEP ;
l'élément endoprothèse vasculaire (102) comprend plusieurs anneaux, chaque anneau ayant un motif sensiblement sinusoïdal et comportant plusieurs sommets (214) ;
l'élément d'orientation (634) comprend plusieurs nervures (636) ;
dans lequel l'étape d'orientation de l'élément endoprothèse vasculaire (102) par rapport à l'élément greffe (104) consiste à disposer l'élément endoprothèse vasculaire (102) par rapport à l'élément d'orientation (634) de sorte qu'un ou plusieurs sommets (214) de l'élément endoprothèse vasculaire (102) soient en contact avec la ou les nervures (636) de l'élément d'orientation (634) ; et éventuellement
dans lequel l'élément endoprothèse vasculaire (102) comprend du nitinol ; ou
dans lequel chaque nervure des plusieurs nervures (636) a une certaine largeur, la largeur étant sensiblement la même pour toutes les nervures des plusieurs nervures (636); et éventuellement
dans lequel la largeur d'au moins une des nervures des plusieurs nervures (636) est égale à la distance qui sépare deux sommets adjacents (214) de l'élément endoprothèse vasculaire (102).

9. Article de fabrication d'une endoprothèse vasculaire-greffe (100), comprenant :
un mandrin sensiblement tubulaire (630) comprenant une surface extérieure, un axe longitudinal, une fente (632) et un élément d'orientation (634),
dans lequel l'élément d'orientation (634) est situé à l'intérieur de la fente (632) et est sensiblement parallèle à l'axe longitudinal du mandrin tubulaire (630), et peut être déplacé perpendiculairement par rapport à la surface extérieure du mandrin tubulaire (630), et
dans lequel l'élément d'orientation (634) comprend plusieurs nervures (636), et dans lequel la position de chaque nervure des plusieurs nervures (636) concorde avec un motif souhaité d'endoprothèse vasculaire (210).

10. Article selon la revendication 9, dans lequel le motif d'endoprothèse vasculaire (210) comprend un motif sensiblement hélicoïdal ; ou
dans lequel chaque nervure des plusieurs nervures (636) a une certaine largeur, la largeur étant sensiblement la même pour toutes les nervures des plusieurs nervures (636) ; ou
dans lequel chaque nervure des plusieurs nervures (636) a une certaine largeur, la largeur variant entre au moins deux nervures des plusieurs nervures (636).
